# EUROPEAN PATENT APPLICATION

(11) **EP 2 051 076 A1**
(43) Date of publication of application: **22.04.2009**
(21) Application number: 07118769.4
(22) Date of filing: 18.10.2007
(51) Int. Cl.: G01N 33/569, G01N 33/74

(54) **Interaction of probe molecules and cells as a diagnostic marker**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The technical field of the present invention relates to cell-based diagnosis of diseases. In particular, the present invention pertains to the field of diagnosing and/or staging coronary artery diseases based on the interaction of VEGF and monocytes.

## Description

### SUBJECT OF THE INVENTION

The technical field of the present invention relates to cell-based diagnosis of diseases. In particular, the present invention pertains to the field of diagnosing coronary artery diseases.

### BACKGROUND OF THE INVENTION

It is well known that growth factors are involved in the development of a variety of diseases including coronary artery disease, diabetes, and various types of cancer such as e.g. breast cancer.

As a consequence, attempts have been made to elucidate the precise function of growth factors but also other disease-related factors during disease development.

Concurrently, with the increasing knowledge of the role of e.g. growth factors during development of coronary artery diseases or different types of cancers, attempts have been to use these factors as a diagnostic marker, either for ongoing disease development or the future-likely occurrence of the disease in question. One approach typically undertaken in this respect is to determine whether expression of a disease-specific factor such as a growth factor is up-regulated or down-regulated compared to a control individual that is known not to suffer from the disease in question.

While these approaches allowed for significant improvements in the diagnosis and therapy of a multitude of diseases, there is a continuing need for diagnostic methods/test systems that allow a physician to decide on the disease state of a patient.

### OBJECT AND SUMMARY OF THE INVENTION

It is one objective of the present invention to provide novel approaches for diagnosing different types of diseases.

It is a further objective of the present invention to provide methods that allow obtaining data that can then be used in diagnosing a variety of diseases.

These objectives as well as others which will become apparent from the ensuing description are attained by the subject matter of the independent claims. Some of the preferred embodiments of the present invention are defined by the dependent claims.

The present invention in one embodiment relates to a method of diagnosing and/or staging a disease comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule;
e) Deciding on the occurrence and/or the stage of the disease in question based on the data obtained in step d).

Deciding on the occurrence and/or the grade of the disease in step e) may be undertaken by different approaches. Thus, one may repeat steps a) to d) for a patient at different times to establish a pattern for the extent of interaction. If one observes a change in the extent of interaction over time this may be an indication of ongoing disease development.

In addition or alternatively one may compare the data obtained in step d) with a suitable control of healthy subjects. In this embodiment the invention relates to a method of diagnosing and/or staging a disease comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule;
e) Providing at least one control sample of a human or animal being not suffering from the disease in question wherein said control sample comprises at least one cell that is capable of specifically interacting with said at least one probe molecule;
f) Contacting said at least one control sample with said at least one carrier structure;
g) Determining the extent of interaction of said at least one cell within said at least one control sample with said at least one probe molecule;
h) Comparing the extent of interaction as determined in steps d) and g);
i) Deciding on the occurrence, stage or risk of the disease or disease event in question based on the comparison made in step h).

In another object embodiment the present invention relates to a method of data acquisition comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule.

One may use the method of data acquisition again to either monitor a subject over a period of time by repeating steps a) to d) and/or to compare the data with those obtained for a control. In this latter case, the invention relates to a method of data acquisition comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule;
e) Providing at least one control sample of a human or animal being not suffering from the disease in question wherein said control sample comprises at least one cell that is capable of specifically interacting with said at least one probe molecule;
f) Contacting said at least one control sample with said at least one carrier structure;
g) Determining the extent of interaction of said at least one cell within said at least one control sample with said at least one probe molecule;
h) Comparing the extent of interaction as determined in steps d) and g).

Typically said carrier structure may be selected to be a microcarrier structure such as a microcarrier bead having a diameter of about 20 µm to about 1000 µm. A size of about 75 µm to about 350 µm, of about 100 µm to about 200 µm and about 150 µm can be preferred.

The methods in accordance with the invention can be used to diagnose, prognose, and/or stage a variety of diseases, in which angiogenesis is a central physiological process such as coronary artery diseases, diabetes, different types of cancers, inflammatory and autoimmune diseases such as rheumatoid arthritis constituting typical examples. Some of the preferred embodiments relate to the diagnosis of coronary artery diseases, including diseases such as atherosclerosis.

In a typical embodiment of the present invention, the said at least one probe molecule will be selected from the group comprising small molecules, peptides, glycopeptides, proteins, glycoproteins, receptors, receptor ligands, antibodies, growth factors, hormones, cytokines, chemokines, cell adhesion molecules, vitamins, nucleic acids and derivatives thereof, lipids and glycanes. A particularly preferred embodiment may relate to probe molecules which are growth factors such as e.g. vascular endothelial growth factor (VEGF), vascular cell adhesion molecule 1 (VCAM-1).

The cell (type) the association of which with the probe molecule is to be determined will depend to some extent on the disease to be analysed as well as the probe molecules being used. Typical examples of cells that are investigated in embodiments of the present invention include embryonic, fetal or adult stem cells, progenitor cells, blood cells, fibroblasts, tumour cells and urinal cells. In a preferred embodiment, the cells can be derived from blood cells and include monocytes, macrophages, neutrophils, B and lymphocytes, circulating tumor cells, circulating stem cells, circulating fetal cells, basophils and eosinophils.

A particularly preferred embodiment relates to methods of diagnosing and methods of data acquisition as outlined above in which the at least one probe molecule is VEGF, in which the at least one cell (type) which is analysed are monocytes and in which the disease to be diagnosed is arteriolosclerosis or atherosclerosis.

In some embodiments, the extent of interaction of the probe molecule that is associated with a carrier structure and the cell type of the sample will be determined by recording the binding of the cell to the probe molecules. In one of the preferred embodiments of the present invention, determination of the extent of interaction will involve repeating this analysis for different amounts of the same probe molecule being bound to the carrier structure. This will allow to determine for a given sample the extent by which cells of the sample bind to the carrier structures depending on the amount of probe molecule being associated with the carrier structure resulting in a profile of amount of cells/carrier structure versus amount of probe molecule/carrier structure.

In some embodiments of the present invention, these profiles may be compared for a human or animal being suspected of suffering from the disease in question and a control human or animal subject which is known not to suffer from the disease so that a decision as to the ongoing development of the disease or likely future occurrence of the disease is possible on the basis of the profiles described above.

Other embodiments of the present invention will become apparent from the detailed description hereinafter.

### FIGURE LEGENDS

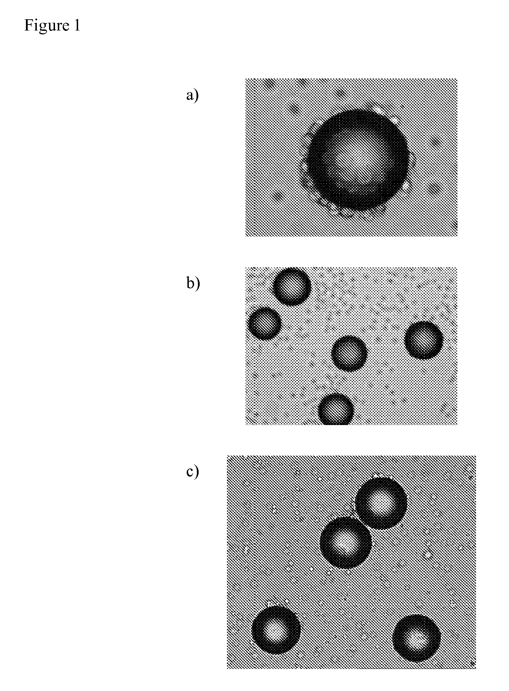

Fig. 1 Depicts adherence of cells to VEGF-functionalized microcarrier beads. a) depicts the association of U937 monoblast cells to microcarrier beads which have been functionalized with 1 ng/mL VEGF. b) depicts control microcarrier beads without VEGF. c) depicts binding of mononuclear cells isolated from whole human blood to microcarrier beads which have been functionalized with 1 ng/mL VEGF.

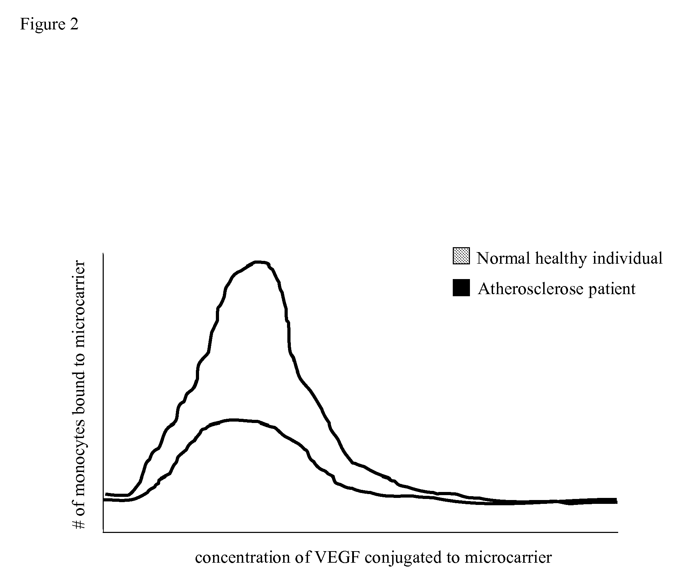

Fig. 2 depicts a hypothetic scenario in which a normal healthy individual shows a different distribution of monocyte binding to VEGF-functionalized microcarrier beads compared to a patient suffering from atherosclerosis. The upper curve shows the binding of the normal healthy individual while the lower curve shows the binding for the atherosclerosis patient.

### DETAILED DESCRIPTION OF THE INVENTION

The inventors of the present invention surprisingly found that one may use the capability of cells such as e.g. monocytes to interact with specific growth factors such as VEGF in the diagnosis of diseases.

Without wanting to be bound to a specific theory, it is assumed that cells within the blood of patients suffering from e.g. atherosclerosis may show a different capacity to interact with their natural ligands such as e.g. VEGF or VCAM compared to the same type of cells that are derived from a healthy individual. This different capacity of certain cell types to interact with their natural ligands or signal mediators, depending on whether a certain disease is in the process of developing may reflect an altered capacity of the cells themselves or relate to e.g. an increased or reduced expression of the signal mediator to which the cells usually bind. The inventors of the present invention have set out to use the above described findings in a simple and straightforward assay to determine the association behaviour of cells with probe molecules which are known to be involved in a specific disease and to then use the extent of association observed as a diagnostic and/or prognostic marker.

Before some of the embodiments of the present inventions are described in more detail, the following definitions are introduced.

As used in this specification and in the appended claims, the singular forms of "a" and "an" also include the respective plurals unless the context clearly dictates otherwise. Thus, the term "a probe molecule" can include more than one probe molecule, namely two, three, four, five etc. probe molecule.

The terms "about" or "approximately" in the context of the present invention denotes an interval of accuracy that the person skilled in the art will understand to still ensure the technical effect of the feature in question. The term typically indicates deviation from the indicated numerical value of +/- 10%, and preferably +/- 5%.

It is to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to disclose a group which preferably consists only of these embodiments.

Further definitions of terms will be given in the context of which the terms are used.

As has been mentioned above, the invention relates in one embodiment to a method of diagnosing or staging a disease comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule;
e) Deciding on the occurrence and/or stage of the disease in question based on the data obtained in step d).

As mentioned above, one may repeat steps a) to e) for a patient at different times to establish a pattern for the extent of interaction. If one observes a change in the extent of interaction over time this may be an indication of ongoing disease development.

In addition or alternatively one may compare the data obtained in step d) with a suitable control of healthy subjects. In this embodiment the invention relates to a method of diagnosing and/or staging a disease comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule;
e) Providing at least one control sample of a human or animal being not suffering from the disease in question wherein said control sample comprises at least one cell that is capable of specifically interacting with said at least one probe molecule;
f) Contacting said at least one control sample with said at least one carrier structure;
g) Determining the extent of interaction of said at least one cell within said at least one control sample with said at least one probe molecule;
h) Comparing the extent of interaction as determined in steps d) and g);
i) Deciding on the occurrence, stage or risk of the disease or disease event in question based on the comparison made in step h).

In one embodiment all of the aforementioned steps are performed outside the human or animal body.

In another embodiment the invention relates to a method of data acquisition comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule.

In one embodiment, one may repeat steps a) to d) for a patient to establish an image of how the data observed change over time.

Alternatively and/or additionally the invention also relates to a method of data acquisition comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule;
e) Providing at least one control sample of a human or animal being not suffering from the disease in question wherein said control sample comprises at least one cell that is capable of specifically interacting with said at least one probe molecule; Contacting said at least one control sample with said at least one carrier structure;
f) Determining the extent of interaction of said at least one cell within said at least one control sample with said at least one probe molecule;
g) Comparing the extent of interaction as determined in steps d) and g).

In one embodiment all of the aforementioned steps are performed outside the human or animal body.

These methods will be discussed in conjunction hereinafter with respect to their specific embodiments.

The carrier structures to be used in the present invention will typically take a 3-dimensional form as this allows the carrier structures to make an intimate contact with the cells that are present within the sample and that should be detected using the methods in accordance with the invention.

The microcarrier structures may have any form that is suitable for the purposes of the present invention. They may thus have a rectangular shape, a triangular shape etc. Beads with round or substantially round form can be preferred according to the present invention. Thus, the carrier structures of the present invention in a preferred embodiment will typically have a round, elliptical or spheroid form.

While the size of the carrier structures is not critical, it can be preferred to use carrier structures that are in the micron size range. These carrier structures will be designated as microcarrier structures. Typically, microcarrier structures will have a diameter in the range of approximately 20 µm to about 1000 µm. Diameters in the range of approximately 75 µm to approximately 300 µm may be preferred, as may be diameters in the range of approximately 100 µm to about 200 µm. However, diameters in the range of 120 µm to about 1800 µm or about 150 µm may also be preferred. If such microcarrier structures take the aforementioned preferred round, elliptical or spheroid form they may be designated as microcarrier beads.

There are no specific requirements to the components or the interior structure of the microcarrier structures or beads except that the structures or beads must be capable of allowing a probe molecule to associate therewith and must be capable of allowing cells to associate with the probe molecules.

With respect to the aforementioned requirements, e.g. microcarrier beads may be produced from materials such as polystyrene, polydivnylbenzene and polymethylmethacrylate and biodegradable polymers such as poly-lactides, polyclycolides, polycaprolacton and copolymers thereof.

Such beads are in principle commercially available from e.g. SoloHill Engineering, Inc (Ann Arbor, Michigan, USA).

The beads may be further modified, e.g. coated in order to ensure adherence of probe molecules and/or to allow for association of a certain type of cell (see below).

The person skilled in the art is familiar with the production of microcarrier beads. Thus, one may obtain these beads from aforementioned commercial sources or produce them according to the protocols being described in e.g. Gu et al (J. Control Release (2004), 96.3:463-472), Li et al (Acta Pharmacol. Sin. (2006) 27.6:754-759), Norrgren et al (Dev. Biol. Stand. (1983), 55:43-51) or Tatard et al (Biomaterials (2005), 26.17:3727-3737). Thus, the bead technology to create both e.g. microcarrier beads as well as probe covered-beads is known to the skilled person.

Microcarrier beads can e.g. be synthesized e.g. by controlled emulsification techniques such as submerged ink-jet printing.

If a microcarrier bead is to be produced from a polymer such as polystyrene, a polymer solution in a solvent such as dichloromethane can be ink-jetted into an aqueous phase containing a stabilizer for the monodisperse emulsion that is formed. To this end, one can use e.g. polyvinyl alcohol but also proteins could be used to achieve efficient stabilization. Subsequently polymer beads with a narrow size distribution are formed upon removal of the solvent.

If one intends to incorporate e.g. peptides/proteins or nucleic acids as probes which can be water soluble, a double emulsion technique can be used. To this end, the water-soluble peptides and/or proteins may be first dissolved in an aqueous phase and this aqueous phase is emulsified in the polymer solution. If necessary or desired, additional stabilizers such as e.g. Pluronic can be added. This first emulsion is then ink-jetted into a second aqueous phase and beads are again formed upon removal of the solvent.

As has been mentioned above, the methods in accordance with the present invention rely on carrier structures and preferably microcarrier structures or beads which are associated with at least one probe molecule.

Such a probe molecule may be any type of molecule that is capable of specifically interacting with a certain cell type. Typically, probe molecules by their native function may act as a ligand for a cell-surface receptor on the cells to detected.

Thus, probe molecules may be selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycanes.

Growth factors and cell adhesion molecules may be preferred as probe molecules. Typical growth factors include e.g. human growth factor, human nerve growth factor, platelet derived growth factor, TGFβ, EGF, VEGF, TNF-a,TNF-b, FGFs, TGF-a, Epo, IGF-I, IGF-II, IL-1, IL-2, IL-6, IL-8, INF-g and CSFs as well as integrins and CAMs including VLA-1 to VLA-6, LPAM-1, LFA-1, CR3, CR4, leukointegrin, collagen, laminin, fibronectin, VCAM-1, MAdCAM-1, E-cadherin, ICAM-1, ICAM-2, ICAM-3, C3b, C4b. Other growth factors include CCL 1-28, CXCL 1-16, XCL1, XC:2, CX3CL1, IL 1-22, INF α, β and y, M-CSF, G-CSF, GM-CSF, MIF, any recognized CD marker of hematopoetic cells (CD 1-339), PDGF, NGF, TPO, GDF-8, GDF-9, bFGF, HGF, FGF, DII4 and extracellular matrix components such as collagen, fibronectin and laminin.

A particularly preferred growth factor will be VEGF. A particularly preferred cell adhesion molecule will be vascular endothelial cell adhesion molecule-1 (VCAM-1).

The person skilled in the art will also be aware that fragments (both synthetic peptide and proteolytically cleaved fragments) of the aforementioned molecules can be used as probe molecules as long as such fragments provide the necessary binding specificity.

The association of the at least one carrier structure and at least one probe molecule may be achieved by covalent or non-covalent interaction between the carrier structure and the probe molecule. In case of a non-covalent interaction, it is nevertheless intended that the carrier structures in accordance with the present invention do not significantly release the probe molecules during the time periods over which the methods in accordance with the invention are typically performed. Thus, it is currently not envisaged that the carrier structures should release the probe molecules to e.g. establish a signal gradient of the probe molecule. Rather, a substantially permanent association of the probe molecule and the carrier structure is intended.

In one of the preferred embodiments, the probe molecule may be covalently attached to the carrier structure. This may be achieved by functionalizing e.g. the surface of the carrier structures which preferably are microcarrier structures or microcarrier beads. In the following, functionalization of carrier structures will be discussed mainly with respect to microcarrier beads. However, the person skilled in the art understands that similar principals will also apply to microcarrier structures and other carrier structures as described above.

A person skilled in the art is familiar how to functionalize e.g. the surface of a microcarrier bead with the aforementioned probe molecules.

To this end, one may e.g. coat the microcarrier bead with a self-assembling polymer. Such a self-assembling polymer may e.g. be polyethylenglycol. The polymers may be further functionalised to introduce chemical functionalities that allow e.g. covalent coupling of an antibody to the surface of the microcarrier bead.

If e.g. growth factors or cell adhesion molecules such as VEGF and VCAM-1 are used as a probe and should be covalently coupled to the microcarrier beads, beads may be used that provide functional groups such as carboxyl groups, amine groups, hydroxyl groups, sulfhydryl groups etc. These groups may then be cross-linked either directly to the growth factors or cell adhesion molecules such as VEGF and VCAM-1 or using a linker that may be homo-or hetero-bifunctional.

Thus, micorcarrier beads may be activated for providing a chemical linkage to the probe molecules by e.g. coating the beads with a polymer having e.g. acyl fluoride functionalities. Other covalent attachment chemistries include but are not limited to anhydrides, epoxides, aldehydes, hydrazides, acyl azides, aryl azides, diazo compounds, benzophenones, carbodiimides, imidoesters, isothiocyanates, NHS esters, CNBr, maleimides, tosylates, tresyl chloride, maleic acid anhydrides and carbonyldiimidazole. It should be noted that often the commercially available beads already have a collagen coating, (or other protein). In those cases one can cross link probe molecules such as VEGF and VCAM to the collagen surface by e.g. bifunctional linkers.
As has been mentioned above, a particular preferred type of probe molecule that can be used to modify a microcarrier bead are molecules that activate adhesion of a specific cell type. Thus, in a particularly preferred embodiment, the present invention makes use of microcarrier beads, which are modified to display VEGF and/or VCAM-1. The function of VEGF has been mentioned above. The cell adhesion molecule VCAM-1 is known to specifically bind leukocytes, thus allowing for separation of white blood cells from plasma, platelets and red blood cells in whole blood samples.
The person skilled in the art will be able to identify further suitable probe molecules that are known to detect e.g. specifically a certain cell type. A person skilled in the art will furthermore be capable of identifying suitable attachment chemistry for either covalently or non-covalently associating such a probe molecule with microcarrier beads.

For functionalising of microcarrier beads with e.g. VEGF or VCAM-1, one can rely on typical polystyrene beads of approximately 150 µm diameter. VEGF or VCAM-1 can then be coupled to the surfaces of such beads, which have been functionalised with a reactive group such as e.g. succinimide esters. The succinimide esters will preferably react with primary amines within VEGF or VCAM-1 to form a covalent linkage.

If such microcarrier beads are suspended in a mixture with whole blood or other monocytes and lymphocytes-containing mixtures, this will result in the specific binding of e.g. monocytes and monocytes to the surface of the functionalised microcarrier surfaces depending on the nature of the probe molecule.

As pointed out above, at least one carrier structure which is associated with at least one probe molecule may be contacted with at least one cell.

In the context of the present invention the term "cell" typically refers to cell types that are known to be involved in the development of certain diseases such as coronary artery diseases, cancer, inflammatory diseases, diabetes etc. Cells in accordance with the invention will typically be activated by specific cellular factors during disease development to migrate and/or adhere to certain cellular factors. Typically, the cells will be recruited or repelled by a specific cellular factor during the development of the disease to the site of the disease to exert their function at this site. Thus, the migratory behaviour of cells may differ for e.g. blood samples derived from patients vs healthy subjects.

It is known that e.g. vascular endothelial growth factor is involved in angiogenesis. More specifically, endothelial cells in the arterial wall secrete VEGF which then acts as a signal diffusing away from the site of excretion and establishing a gradient that diminishes as one moves away from the endothelial cells. This VEGF gradient acts on other endothelial cells as well as on cells being present in the blood such as monocyte cells. Typically, the monocytes will respond to VEGF gradients by moving towards increasing concentration of VEGF signals. After the monocytes have adhered to the endothelial cells greeting VEGF, they can start a plethora of signal mechanisms that ultimately result various responses including angiogenesis, inflammation, cell differentiation and phagocytosis.

It has further been observed that recruiting of blood cells including monocytes contribute to the development of coronary artery diseases as well as of the metastasis and angiogenesis of cancer tumors.

Cells in accordance with the invention may thus be selected from the group comprising embryonic, fetal or adult stem cells, progenitor cells, blood cells, fibroblasts, tumour cells and neuronal cells. For the purposes of the present invention, the use of blood cells is preferred. Typically these blood cells comprise B and T lymphocytes, monocytes, macrophages, neutrophils, basophils and eosinophils.

As has been mentioned above, the methods in the present invention may be used in the context of diagnosing and staging a disease. As also pointed out above in the context of the cells to be analyzed by the methods in accordance with the invention, the diseases to be diagnosed may be diseases for which an implication of a specific cell type is known and which are characterised in that the specific cell type is recruited during disease development by a specific factor that may be used in the context of the present invention as the probe molecule.

Typically, diseases in the context of the present invention include e.g. coronary artery diseases such as atherosclerosis and arteriolosclerosis, diabetes, inflammatory diseases such as rheumatoid arthritis, and different types of cancers such as colorectal, lung, prostate and breast cancer. Other cancers may include leukemia, lymphoma, head and neck cancer, non-small cell lung cancer, ovarian cancer, urinary bladder cancer, kidney cancer, cervical cancer etc.

Particularly for coronary artery diseases such as atherosclerosis and certain types of cancer, it is known that blood cells such as monocytes and macrophages play a major contributory role during disease development. For example in atherosclerosis, monocytes are recruited by cellular factors such as VEGF and VCAM-1 and then invade into the epithelium. By complex mechanisms and cholesterol uptake the monocytes ultimately turn into foam cells which contribute to plaque progression, instability and rupture. Similarly, in certain types of cancer, tumour formation is characterised by an aberrant increase of angiogenic activity that is necessary in order to ensure that the tumour receives sufficient blood supply.

In a preferred embodiment, the above described methods in accordance with the invention are thus used for the diagnosis of coronary artery diseases such as atherosclerosis and cancers such as solid tumors by determining the difference in the extent of interaction of blood cells derived from a patient an healthy individual with probe molecules such as cell adhesion molecules and growth factors including VCAM-1 and VEGF.

As mentioned above, the present invention in a preferred embodiment relates to the use of microcarrier beads which comprise probe molecules being growth factors or cell adhesion molecules such as VEGF and VCAM-1 in the context of diagnosing coronary artery diseases or certain types of cancer.

In the following, some specific aspects of the methods in accordance with the invention will be discussed with respect to this preferred embodiment. The person skilled in the art will, however, be aware, that other embodiments of the invention that deal with different probe molecules, different carrier structures, different cell types and different diseases may also be performed along the lines discussed hereinafter.

Typically one will use the microcarrier beads that have been functionalized with a probe molecule such as growth factors or cell adhesion molecules and contact those microcarrier beads with a sample derived from a human or animal being which is suffering from the disease in question. A preferred embodiment is to use a sample that is derived from the blood of a human or animal being, particularly if the probe molecules aim at detecting blood cells such as monocytes or macrophages.

The person skilled in the art knows how to prepare and obtain such samples. For example, one may draw blood from the patient and perform a buffy-coat centrifugation to isolate mononuclear cells resulting in a mixture of lymphocytes and monocytes. As to whether and to what extent pre-purification of the sample is necessary depends on the type of cells to be analysed as well as on the type of disease to be diagnosed.

Once the sample has been prepared it is contacted with the microcarrier structures that have been functionalised with the probe molecules being mentioned above to allow for association between the cells within the sample that are to be detected and the probe molecules. The contacting or incubation step may typically take from about 1 min to about 240 min, from about 5 to about 120, from about 10 to about 60 min, or about 15, 20, 25 and 30 min. A typical incubation range may be about 20 to about 30 min. The use of microcarrier beads is particularly preferred in this context as they allow efficient and rapid achievement of an intimate contact between the probe molecules such as growth factors and the cells within the sample that are to be detected.

Following the contacting step, one determines the extent of interaction between the cells within the sample and the probe molecules. This may be undertaken simply by e.g. light microscopy. Further more elaborate methods of detection are, of course, also available. A person skilled in the art is familiar with detecting cells that have associated with the beads as well to determine the amount of cells that have associated with the beads. For example one may isolate the beads simply by centrifugation and then release the adhered cells by treating the cells in a high salt buffer and counting the cells.

In another embodiment one may use labelled probe molecules. Such labels may e.g. be fluorescent dies that change their absorption and emission characteristics depending on the amount of cells that have adhered to the microcarrier beads. After a normalisation, simple recording of a spectrum may be sufficient to determine the amount of cells that have adhered to the microcarrier beads.

Once one has determined the amount of cells that have interacted with the probe molecule and functionalised microcarrier beads, one can repeat this exercise with a sample obtained from a human or animal individual that is known not to be afflicted by the disease.

Comparing the interaction extent one can then evolve a profile that is characteristic for a certain disease and even a certain stage of a specific disease. To this end one may perform the above described methods as a multi-factorial or analysis.

In a preferred embodiment of such an approach, one will use carrier structures that comprise different amounts of the probe molecules and contact them under otherwise identical parameters with the same samples. Using this approach it will be possible to develop a profile that shows the number of cells that a have adhered to a carrier structure versus the concentration of probe molecule per carrier structure. The profiles obtained for samples that are derived from a patient versus samples that are derived from healthy individuals can then be compared and a statistical analysis may be used to decide on the presence and the state of the ongoing disease development.

The concentration range of the probe molecule to be used on carrier structures in accordance with the invention will depend on the specific probe molecule in question. For VEGF the concentration range will be in the range from about 0.01 ng/mL to 1mg/mL. In these preferred aspects of the embodiment, one thus hinges on e.g. the levels of expression of VEGF and the ability of the patient's monocytes to respond to those levels which are individual and disease-state dependent. Prefered levels will be 1 - 20 ng/mL By testing a range of VEGF concentrations at a constant concentration of blood cells, trends may be elucidated that may not be evident using other approaches.

The advantages of the present invention pertain *inter alia* to the ease of the time and cost effectiveness of the tests. Usually, the migratory behaviour of a cell is determined using approaches such as the Dunn chamber or the Boyden chamber test. These tests, however, including sample preparation and data analysis will usually require at least 6 hours and the necessary time may easily amount to 8 to 10 hours. The methods in accordance with the invention however can be used on a much shorter time scale.

Typically for example, if VEGF is used as the probe molecule and if monocytes are analysed, the incubation time may be as little as 10 to 30 minutes.

While the above invention has been described with respect to some of its preferred embodiments, this is in no way to limit the scope of the invention. The person skilled in the art is clearly aware of further embodiments and alterations to the previously described embodiments that are still within the scope of the present invention.

### EXAMPLES:

### Example I

Microcarrier were commercially obtained from Solohill (www.solohill.com). Subsequently, VEGF was coupled at a concentration of 1 ng/mL to the Solohill microcarrier beads using standard protein cross linking. First the collagen coating on the beads was activated to form a reactive NHS-ester, which was then subsequently reacted with amine groups found on the N-terminal and lysine residues of the VEGF. As a control, a blank microcarrier bead without any coupling of VEGF was used.

Subsequently 10 µL of 1 mg/mL microcarrier beads were reacted for 30 minutes with 2.5 x 10⁶ cells/mL of U937 cells.

Figure 1(a) and (b) show that only in case of VEGF functionalization the U937 cells adhere to the microcarrier beads.

In a second experiment the same microcarrier beads were tested for binding to mononuclear cells which had been isolated from whole blood by standard Ficoll paque centrifugation to isolate mononuclear white blood cells directly from whole blood. Also in this case it was possible to detect a binding of the cells from the sample to the VEGF functionalized beads.

### Hypothetical Example 2

In this hypothetic example it is assumed that the monocytes of a patient suffering from atherosclerosis show a different interaction potential with VEGF than those obtained from a healthy human individual. If one determines the capacity of monocytes for a given sample, e.g. a given cell number to bind at a specific time interval, e.g. 30 minutes to microcarrier beads as they have been described in Example 1, at different concentrations of course, VEGF, it may be possible to obtain a profile that is characteristic for the healthy human individual or the patient suffering from atherosclerosis (see Figure 2).

## Claims

1. Method of diagnosing and/or staging a disease comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule;
e) Deciding on the occurrence and/or stage of the disease based on the data obtained in step d).

2. Method of data acquisition comprising at least the following steps:
a) Providing at least one carrier structure wherein said carrier structure is associated with at least one probe molecule being capable of specifically interacting with at least one cell;
b) Providing at least one sample of a human or animal being suffering from the disease in question wherein said sample comprises at least one cell that is capable of specifically interacting with said at least one probe molecule;
c) Contacting said at least one sample with said at least one carrier structure;
d) Determining the extent of interaction of said at least one cell within said at least one sample with said at least one probe molecule.

3. Method according to claim 1,
wherein said disease is selected from the group comprising coronary artery diseases, cancer, diabetes, autoimmune and inflammatory diseases/disorders..

4. Method according to any of claims 1 to 3,
wherein said at least one carrier structure is a microcarrier bead.

5. Method according to any of claims 1 to 4,
wherein said at least one probe molecule is selected from the group comprising small molecules, peptides, glycopeptides, proteins, glycoproteins, receptors, receptor ligands, antibodies, growth factors, hormones, cytokines, chemokines, cell adhesion molecules, integrins, vitamins, nucleic acids and derivatives thereof, lipids and glycanes.

6. Method according to any of claims 1 to 5,
wherein said at least one cell is selected from the group comprising embryonic, fetal or adult stem cells, progenitor cells, blood cells, fibroblasts, tumour cells and neuronal cells.

7. Method according to claim 6,
wherein said at least one cell is selected from the group of blood cells comprising monocytes, T and B lymphocytes, macrophages, eosinophils, basophiles and neutrophils.

8. Method according to any of claims 1 to 7,
wherein said at least one probe molecule is VEGF, wherein said at least one cell is selected from monocytes and wherein the disease to be diagnosed is atherosclerosis.

9. Method according to any of claims 1 to 8,
wherein said extent of interaction is determined by measuring, for a given sample and/or a given time period, the amount of cells that associate with said at least one carrier structure depending on the amount of said at least one probe molecule that is associated with said at least one carrier structure.
